# EUROPÄISCHE PATENTSCHRIFT

(11) **EP 4 352 152 B1**
(45) Veröffentlichungstag und Bekanntmachung des Hinweises auf die Patenterteilung: **01.01.2025**
(21) Anmeldenummer: 22734244.1
(22) Anmeldetag: 09.06.2022
(51) Int. Cl.: C08L 5/04, A61K 9/06, A61K 47/36, A61P 1/16, A61P 13/04, A61P 41/00, C08J 3/075

(54) **STERILISIERTE MEHR-KOMPONENTEN-ZUSAMMENSETZUNG ZUR ENTFERNUNG VON PARTIKELN**
STERILIZED MULTICOMPONENT COMPOSITION FOR REMOVAL OF PARTICLES
COMPOSITION STÉRILISÉE À PLUSIEURS CONSTITUANTS POUR L'ÉLIMINATION DE PARTICULES

(30) Priorität: 09.06.2021 DE 202021103106 U
(43) Veröffentlichungstag der Anmeldung: 17.04.2024
(73) Patentinhaber: Purenum GmbH, 28359 Bremen (DE)
(72) Erfinder: GRUNWALD, Ingo, 28865 Lilienthal (DE); STÖßLEIN, Sebastian, 28209 Bremen (DE)
(74) Vertreter: Eisenführ Speiser
(86) Internationale Anmeldenummer: PCT/EP2022/065643
(87) Internationale Veröffentlichungsnummer: WO 2022/258734

(56) Entgegenhaltungen:
- EP-B1- 2 747 760
- WO-A1-2014/173468

## Beschreibung

Die vorliegende Erfindung betrifft eine sterilisierte, gelbildende Mehr-Komponenten-Zusammensetzung umfassend eine Komponente enthaltend mindestens ein vernetzbares Polymer und eine Komponente enthaltend mindestens ein Vernetzungsmittel. Weiter betrifft die vorliegende Erfindung eine solche Zusammensetzung bzw. ein solches Gel zur Anwendung in einem Verfahren zum Entfernen von unerwünschten Partikeln aus einem Patienten, sowie ein Verfahren zur Herstellung einer solchen Zusammensetzung und eine Zusammensetzung herstellbar oder hergestellt durch ein solches Verfahren.

Weitere Aspekte der vorliegenden Erfindung oder in deren Zusammenhang, sowie bevorzugte Ausführungsformen sind nachfolgend sowie in den beigefügten Ansprüchen beschrieben.

Die Anhäufung von unerwünschten Ausfallprodukten oder Ablagerungen diverser Art sind ein häufig auftretendes Problem im menschlichen Körper. Bekannte Formen hiervon sind unter anderem Galleinsteine oder Nierensteine. Galleinsteine treten in etwa 10 bis 15 % der erwachsenen Bevölkerung auf, wobei die westlichen Industrieländer besonders betroffen sind. Etwa die Hälfte der deutschen Bevölkerung im Alter von über 60 Jahren hat Gallensteine. Während Gallensteine oftmals asymptomatisch bleiben, kommt es jedoch in einigen Fällen zu Koliken mit starken Schmerzen, erhöhten Leberwerten und allgemeinen Krankheitssymptomen bis hin zur Gelbsucht (Ikterus).

Aus epidemiologischer Sicht stellt das Nierensteinleiden eine der häufigsten Erkrankung der Menschheit dar, deren Inzidenz in Deutschland im Jahr 2000 1,45 % betrug, was wiederum ca. 1.200.000 Neuerkrankungen pro Jahr entspricht. Allein in Deutschland kann insgesamt von ca. 750.000 Behandlungsfällen pro Jahr ausgegangen werden. Die Zahl der Behandlungen zur Steinentfernung in Deutschland wird auf ca. 400.000 / Jahr, davon ca. die Hälfte für Behandlungen von Rezidivsteinen, geschätzt. Die genannten Zahlen lassen sich weltweit auf die millionenfache Durchführung solcher Behandlungen extrapolieren. Mit einer Summe von über 1,5 Milliarden Euro stellt die Nierensteinerkrankung also einen erheblichen Kostenfaktor im deutschen Gesundheitswesen dar. Nierensteine können ebenfalls für starke Schmerzen sorgen, eine Nierenentzündung auslösen, die Nieren schädigen oder gar zu einem (in der Regel einseitigen) akuten Nierenversagen führen.

Die Ursachen beider Problematiken sind vielseitig und reichen von ungesunder, fettreicher Ernährung bzw. Flüssigkeitsmangel oder Bewegungsmangel über Erkrankungen wie Diabetes mellitus oder Gicht bis hin zu genetischer Veranlagung.

Häufig werden Gallen- oder Nierensteine erst in einem bereits fortgeschrittenen Stadium entdeckt, in der Regel dann, wenn die ersten (schwerwiegenderen) Symptome auftreten. Meist sind die entstandenen Steine dann bereits zu groß, als dass diese durch minimalinvasive Verfahren am Stück aus dem Körper entfernt werden können. In diesem Fall werden die entstandenen Steine zertrümmert und die einzelnen Fragmente davon entfernt. In einigen Fällen bilden sich auch mehrere Steine, welche ebenfalls vollständig entfernt werden müssen.

Eine Methode zur Behandlung von Nierensteinen durch gezieltes Auflösen der Ablagerungen unter Verwendung von quartären Ammoniumsalzen wird beispielsweise in US 5,244,913 beschrieben.

Nicht nur im Fall von Gallen- oder Nierensteinen oder weiterer Steine im Körper müssen mehrere unerwünschte Partikel aus einem Patienten entfernt werden. So ist auch in anderen Fällen, beispielsweise bei Trümmerbrüchen, hier Splitter des körpereigenen Knochens, oder Schürfwunden, hier beispielsweise Fremdkörper wie Steine, Metall-, Kunststoff- oder Holzsplitter bzw. Fragmenten davon, eine Vielzahl an Partikeln möglichst vollständig aus dem Patienten zu entfernen.

All diese Fälle haben gemein, dass eine Vielzahl an Partikeln möglichst unkompliziert, gleichzeitig aber auch möglichst vollständig aus dem Patienten entfernt werden muss.

Verlassen beispielsweise die Gallen- oder Nierensteine den Körper nicht auf natürlichem Wege oder bestehen medizinische Indikationen zur sofortigen Therapie, stellt die Endoskopie (minimalinvasive Spiegelungstechniken) neben der extrakorporalen Stoßwellenbehandlung (ESWL) den therapeutischen "Gold-Standard" dar. Angesichts der zunehmenden Evidenz für schlechtere Ergebnisse der ESWL, werden endoskopische Verfahren bevorzugt angewendet. Es ist davon auszugehen, dass aktuell 60-70 % aller Steinpatienten endoskopisch behandelt werden. Diese Tendenz ist steigend. Unter Zuhilfenahme endoskopischer Techniken werden Nierensteine vor Ort zerkleinert und entfernt. Ein bisher ungelöstes Problem stellen insbesondere kleine Restfragmente (<2 mm) dar, die während der Behandlung nicht effektiv entfernt werden können. Verbliebene Nierensteinfragmente fungieren als "Kristallisationskeime" aus denen sich zu 70 % neue Steine entwickeln. Dies wiederum führt zu erneuten medizinischen Problemen und Behandlungsbedarf. Solche Fragmente wurden früher als klinisch irrelevante Restfragmente (CIRF) bezeichnet, wobei sich aber in den letzten Jahren deutlich zeigte, dass diese sehr wohl klinisch relevant sind.

Bei der Lithotripsie werden Nierensteine durch extrakorporale Stoßwellen oder endoskopisch eingeführte Laser- oder Druckluftsonden zertrümmert. Dabei entstehen unterschiedlich große Bruchstücke, die entweder mit Hilfe von Fassinstrumenten entfernt oder ausgespült werden können. Ein bei der Lithotripsie auftretendes Problem ist, dass sich die Fragmente während des Zertrümmerns verteilen können oder in schwer zugängliche Regionen gelangen.

WO 2005/037062 betrifft eine Methode, mit der Nierensteine mit Hilfe eines Polymerpfropfens in einem bestimmten Bereich eingeschlossen (nicht umschlossen) werden, wodurch Gewebeschäden durch die entstehenden Fragmente während der Zertrümmerung weitgehend verhindert werden können. Gemäß WO 2005/037062 wird auf zumindest einer Seite eines Nierensteins eine gelbildende Flüssigkeit in das Lumen injiziert, beispielsweise ein thermosensitives Polymer, welches bei Körpertemperatur einen Gelpfropfen bildet. Das Polymer kommt dabei in der Regel nicht in Kontakt mit dem Nierenstein, dient aber dazu, die Effizienz der Lithotripsie zu erhöhen, indem es ein Verschieben des Nierensteins verhindert, und das umgebende Gewebe vor Schädigung durch die Fragmentierung schützt. Eine Anwendung dieses Systems wird explizit nur außerhalb der Niere empfohlen bzw. erlaubt.

Ein Ansatz um Objekte, wie beispielsweise Blutgerinnsel, unter Verwendung eines Klebstoffes aus dem Körper zu entfernen, wird in US 2008/0065012 angegeben. Der Klebstoff wird dabei auf einer Oberfläche verteilt und mit Hilfe eines Katheters in den Körper eingeführt. Wenn das Objekt an der Oberfläche angehaftet ist, wird der Katheter wieder herausgezogen und nimmt das Objekt mit.

Auf biologischen Makromolekülen basierende Klebstoffe und insbesondere gelbildende Polymer-Systeme finden in der Medizintechnik zunehmend Anwendungen. Dabei ist ihre hohe Biokompatibilität eines der wichtigsten Auswahlkriterien.

In US 6,663,594 B2 wird ein Verfahren zur Immobilisation eines Objekts, beispielweise eines Nierensteins, im Körper beschrieben, bei dem eine gelbildende Flüssigkeit in den Körper injiziert wird. In Kontakt mit dem Objekt wird ein Gel gebildet, welches das Objekt zumindest teilweise erfasst und immobilisiert. Die Immobilisierung dient dazu, das Objekt anschließend fragmentieren zu können, ohne eine Verteilung der Bruchstücke zu riskieren bzw. um das Objekt bzw. die Bruchstücke mit einem endoskopischen Werkzeug aus dem Körper zu entfernen. Dabei verhindert das Gel, dass das Objekt bzw. ein Bruchstück verrutscht und von dem Werkzeug nicht erfasst werden kann. Nach Entfernen des Objekts bzw. der Bruchstücke wird das Gel aufgelöst oder mittels eines endoskopischen Werkzeugs extrahiert. Nachteil dieser Methode ist, dass beim Zertrümmern der Nierensteine das bereits abgebundene Gel zerstört werden kann und dadurch wieder Fragmente freigesetzt werden können, oder dass einzelne Fragmente aus dem Polymer austreten. Ein Greifen der Fragmente im Gel ist mit den herkömmlichen Greifwerkzeugen nicht möglich. Zudem ist die beschriebene Prozedur sehr aufwendig, da die Steine oder Stein-Fragmente einzeln gefasst und entfernt werden. Im Ergebnis bleiben mit relativ großer Wahrscheinlichkeit einzelne Steinfragmente zurück.

Ein Problem bei der Lithotripsie ist insbesondere das Auftreten mittelgroßer Steinfragmente (insbesondere < 2 mm), auch als "Gries" bezeichnet, da diese Bruchstücke weder effizient gegriffen noch gespült werden können. Restfragmente dieser Größe rutschen durch die Maschen der Greifinstrumente (Fasszängchen oder -körbchen) und machen die Extraktion von Gries sehr zeitaufwändig und bei größeren Steinmassen praktisch undurchführbar. Das Zurückbleiben solcher Nierensteinfragmente führt jedoch in einem sehr hohen Prozentsatz der Fälle zur Bildung neuer Nierensteine, da die Bruchstücke bzw. Fragmente als "Kristallisationskeime" fungieren.

WO 2014/173467 beschreibt ein gelbildendes System, umfassend eine Komponente enthaltend ein oder mehrere vernetzbare Polymere, eine Komponente enthaltend ein oder mehrere Vernetzungsmittel und gegebenenfalls eine Komponente enthaltend magnetisierbare Teilchen. Das gelbildende System kann zur Entfernung von Harnsteinen eingesetzt werden. Dabei wird / werden der / die Harnsteine zertrümmert. Anschließend werden die Komponente enthaltend ein oder mehrere Vernetzungsmittel und die Komponente enthaltend magnetisierbare Teilchen gemischt und per Katheter über ein Endoskopgerät in den Bereich des Harntrakts, der die Fragmente des bzw. der zertrümmerten Harnsteine(s) enthält, injiziert. Daraufhin wird die Komponente enthaltend ein oder mehrere vernetzbare Polymere zugegeben, wodurch sich ein Gel bildet. Das gefestigte Gel umschließt dabei teilweise oder vollständig die Harnsteine bzw. die Fragmente davon und kann mittels eines Greifinstruments über das Operationsendoskop mitsamt den Harnsteinen bzw. den Fragmenten davon entfernt werden.

WO 2014/173468 beschreibt ein Kit oder eine Mehr-Komponente Zusammensetzung umfassend eine Komponente (A) enthaltend ein oder mehrere vernetzbare, vorzugsweise kationisch vernetzbare, Polymer(e), und eine Komponente (B), enthaltend ein oder mehrere Vernetzungsmittel zum Vernetzen des bzw. der vernetzbaren Polymer(e) der Komponente (A).

Die Injektion von Komponenten, die nach erfolgter Mischung ein Gel bilden, weist häufig die Schwierigkeit auf, eine geeignete Balance zu finden zwischen einer ausreichend hohen Viskosität, sodass sich ein Gel besonders leicht bilden kann, und einer ausreichend niedrigen Viskosität, sodass die jeweilige Komponente besonders gut injiziert werden kann. Das entstandene Gel sollte zudem eine geeignete Festigkeit aufweisen, um besonders leicht wieder entfernt werden zu können. Zudem sollte die Zeit zwischen Mischung der Komponenten ausreichend niedrig sein, um beispielsweise einen medizinischen Eingriff möglichst rasch durchführen zu können. Gleichzeitig darf die Zeit aber auch nicht zu kurz sein, da sonst möglicherweise keine ausreichende Durchmischung der Komponenten erfolgt und ein sehr inhomogenes Gel entstehen kann, bzw. Klumpen bei der Gelbildung entstehen können.

Eine weitere Schwierigkeit besteht darin, dass die Komponenten häufig für den medizinischen Einsatz sterilisiert werden müssen. Nach erfolgter Sterilisation weisen die Komponenten bzw. Bestandteile der Komponenten häufig nur noch eine stark verringerte Funktion, insbesondere zur Gelbildung, auf. Gleiches gilt auch für die Lagerung der Komponenten. So werden insbesondere die vernetzbaren Polymere solcher Komponenten während der Sterilisation degradiert, wodurch deren Fähigkeit zur anschließenden Gelbildung stark reduziert wird oder gar ganz verloren geht.

Die primäre Aufgabe der vorliegenden Erfindung war es daher, eine Möglichkeit bereitzustellen, bei welcher Partikel aus einem Patienten entfernt werden können, und bei welcher die oben genannten Vorteile, bzw. möglichst viele dieser Vorteile, ohne die aufgeführten Nachteile, bzw. mit möglichst wenigen der Nachteile, erzielt werden können. Insbesondere war es Aufgabe der vorliegenden Erfindung, ein verbessertes, sterilisiertes Mittel bereitzustellen, dass dazu geeignet ist, Partikel zuverlässig aus dem Körper extrahieren zu können.

Die primäre Aufgabe der vorliegenden Erfindung wird gelöst durch eine sterilisierte, gelbildende Mehr-Komponenten-Zusammensetzung, vorzugsweise Zwei-Komponenten-Zusammensetzung, zur Bildung eines Gels,
die Zusammensetzung bestehend aus oder umfassend
Komponente (A), enthaltend
   (i) ein oder mehrere vernetzbare, vorzugsweise kationisch vernetzbare, Polymer(e), wobei das oder ein Polymer Natriumalginat ist,
   (ii) Wasser,
   (iii) NaCl in einem Bereich von 0,3 bis 1,2 Gew.-%, basierend auf dem Gesamtgewicht der Komponente (A)
   (iv) einen oder mehrere Phosphatpuffer und
   (v) optional: einen oder mehrere Farbstoffe
   und
Komponente (B), enthaltend
   (a) ein oder mehrere Vernetzungsmittel zum Vernetzen des bzw. der vernetzbaren Polymer(e) in Bestandteil (i) der Komponente (A), vorzugsweise wobei das oder ein Vernetzungsmittel CaCl₂ ist, und
   (b) Wasser, sowie
   (c) optional: einen oder mehrere Farbstoffe.

Bevorzugt ist, dass Bestandteil (a) der Komponente (B) CaCl₂ enthält.

Bevorzugt ist, dass Bestandteil (i) der Komponente (A) Natriumalginat und Bestandteil (a) der Komponente (B) CaCl₂ ist.

Natriumalginat ist als vernetzbares Polymer für den Einsatz im Körper besonders geeignet, da es keine inflammatorischen Reaktionen oder Immunabstoßungen hervorruft und ein geringes Risiko eines Gewebetraumas mit sich bringt. Zudem ist es biologisch abbaubar. Die Vernetzung erfolgt zügig aber ohne dabei feine Gewebsstrukturen des Patienten oder die Endoskopieinstrumente zu verkleben. Die entstehenden Gele weisen eine ausreichende Stabilität und Flexibilität auf, um zusammen mit den Partikeln extrahiert werden zu können.

Vorzugsweise weisen mindestens 40 %, bevorzugt mindestens 50 %, bevorzugt mindestens 60 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, bevorzugt mindestens 96 %, bevorzugt mindestens 97 %, bevorzugt mindestens 98 %, bevorzugt mindestens 99 % des Natriumalginats in Komponente (A) eine molare Masse von mindestens 110.000 g/mol, bevorzugt mindestens 125.000 g/mol, bevorzugt mindestens 150.000 g/mol, bevorzugt mindestens 175.000 g/mol, bevorzugt mindestens 200.000 g/mol, bevorzugt mindestens 225.000 g/mol, bevorzugt mindestens 250.000 g/mol, bevorzugt mindestens 275.000 g/mol, bevorzugt mindestens 300.000 g/mol, bevorzugt mindestens 325.000 g/mol, bevorzugt mindestens 350.000 g/mol, bevorzugt mindestens 375.000 g/mol, bevorzugt mindestens 400.000 g/mol auf.

Vorzugsweise weisen mindestens 40 %, bevorzugt mindestens 50 %, bevorzugt mindestens 60 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, bevorzugt mindestens 96 %, bevorzugt mindestens 97 %, bevorzugt mindestens 98 %, bevorzugt mindestens 99 % des Natriumalginats in Komponente (A) eine molare Masse von maximal 700.000 g/mol, bevorzugt maximal 600.000 g/mol, bevorzugt maximal 550.000 g/mol, bevorzugt maximal 500.000 g/mol, bevorzugt maximal 475.000 g/mol, bevorzugt maximal 450.000 g/mol, bevorzugt maximal 425.000 g/mol, bevorzugt maximal 400.000 g/mol, bevorzugt maximal 380.000 g/mol, bevorzugt maximal 360.000 g/mol, bevorzugt maximal 350.000 g/mol, bevorzugt maximal 340.000 g/mol, bevorzugt maximal 320.000 g/mol, bevorzugt maximal 300.000 g/mol, bevorzugt maximal 280.000 g/mol, bevorzugt maximal 260.000 g/mol, bevorzugt maximal 250.000 g/mol, bevorzugt maximal 240.000 g/mol, bevorzugt maximal 220.000 g/mol, bevorzugt maximal 200.000 g/mol auf.

Jegliche Kombination der beschriebenen minimalen und maximalen molaren Masse sind hiermit für jede beschriebene Menge im Alginat offenbart. Gleiches gilt auch für den Fall, dass die minimale und die maximale molare Masse für eine unterschiedliche Menge des Alginats gilt.

Alginat, bzw. Natriumalginat, ist ein Polysaccharid, das aufgebaut ist aus den beiden Uronsäuren Guluronsäure (G) und Mannuronsäure (M). In Alginaten kann der Anteil an G und der Anteil an M im Alginatmolekül stark variieren. Der Anteil an G bestimmt dabei den Anteil an M, d.h. falls der G-Anteil bei 75 % liegt, so liegt der M-Anteil bei 25 %.

Vorzugsweise weisen mindestens 40 %, bevorzugt mindestens 50 %, bevorzugt mindestens 60 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, bevorzugt mindestens 96 %, bevorzugt mindestens 97 %, bevorzugt mindestens 98 %, bevorzugt mindestens 99 % des Natriumalginats in Komponente (A) einen G-Anteil von mindestens 30 %, bevorzugt mindestens 35 %, bevorzugt mindestens 40 %, bevorzugt mindestens 45 %, bevorzugt mindestens 50 %, bevorzugt mindestens 55 %, bevorzugt mindestens 60 %, bevorzugt mindestens 65 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, auf.

Vorzugsweise weisen mindestens 40 %, bevorzugt mindestens 50 %, bevorzugt mindestens 60 %, bevorzugt mindestens 70 %, bevorzugt mindestens 75 %, bevorzugt mindestens 80 %, bevorzugt mindestens 85 %, bevorzugt mindestens 90 %, bevorzugt mindestens 95 %, bevorzugt mindestens 96 %, bevorzugt mindestens 97 %, bevorzugt mindestens 98 %, bevorzugt mindestens 99 % des Natriumalginats in Komponente (A) einen G-Anteil von maximal 99 %, bevorzugt maximal 98 %, bevorzugt maximal 97 %, bevorzugt maximal 96 %, bevorzugt maximal 95 %, bevorzugt maximal 90 %, bevorzugt maximal 85 %, bevorzugt maximal 80 %, bevorzugt maximal 75 %, bevorzugt maximal 70 %, bevorzugt maximal 65 auf.

Jegliche Kombination der beschriebenen minimalen und maximalen G-Anteile sind hiermit für jede beschriebene Menge im Alginat offenbart. Gleiches gilt auch für den Fall, dass der minimale und der maximale G-Anteil für eine unterschiedliche Menge des Alginats gilt.

Ebenfalls offenbart sind hiermit alle Kombinationen aus den beschriebenen minimalen und/oder maximalen molaren Massen und den beschriebenen minimalen und/oder maximalen G-Anteilen im Alginat. Gleiches gilt auch für den Fall, dass die minimale und/oder maximale molare Masse und/oder der minimale und/oder maximale G-Anteil für eine unterschiedliche Menge des Alginats gilt.

CaCl₂ stellt in physiologischen Systemen natürlich vorkommende Kationen bereit, die in Form von biologisch verträglichen Lösungen einfach verabreicht werden können. Sie haben eine geeignete Koordinationschemie und können stabile Chelatkomplexe zur Vernetzung und somit zur Gelbildung ausbilden.

Der Begriff "gelbildende Zusammensetzung", wie hierin verwendet, ist vorzugsweise so zu verstehen, dass die Zusammensetzung nicht nur geeignet ist, ein Gel zu bilden, sondern dass durch Mischung der Komponenten der Zusammensetzung auch tatsächlich ein Gel entsteht.

Das durch Mischen der Komponenten der Zusammensetzung entstehende Gel ist vorzugsweise ein Hydrogel.

Es wurde überraschend gefunden, dass Komponente (A), wie in den erfindungsgemäßen Zusammensetzungen beschrieben, vorteilhaft sterilisiert werden kann, sodass Komponente (A) nach erfolgtem Sterilisationsverfahren weiterhin funktionsfähig ist. So können einzelne bzw. die einzelnen Komponenten oder die Mehr-Komponenten-Zusammensetzung sterilisiert werden, um Anwendung im medizinischen, z.B. invasiven oder operativen, Bereich zu finden, insbesondere um in einen Patienten eingebracht zu werden.

Durch die Kombination aus NaCl und Phosphat(en) in Komponente (A), wie in den erfindungsgemäßen Zusammensetzungen beschrieben, wurde die Verringerung der Gelbildung nach der Sterilisation signifikant reduziert (vgl. Beispiel 3). Überraschend wurde gefunden, dass neben dem Vorhandensein an Phosphaten in Komponente (A) auch die Konzentration an NaCl wichtig ist, um die Gelbildung auch nach erfolgter Sterilisation vorteilhaft zu ermöglichen. Überraschenderweise üben Phosphate sowie NaCl, wie in den erfindungsgemäßen Zusammensetzungen beschrieben, einen synergistischen Effekt auf den Erhalt der Funktion nach Sterilisation aus (vgl. Beispiel 3).

Vorzugsweise liegt die Menge an NaCl in einem Bereich von 0,3 bis 1,2 Gew.-%, besonders bevorzugt in einem Bereich von 0,4 bis 1,0 Gew.-%, basierend auf dem Gesamtgewicht der Komponente (A).

Falls einer oder mehrere der Bestandteile der Komponente (A), neben Bestandteil (iii) NaCl enthalten sollte, wird das gesamte in Komponente (A) vorhandene NaCl zur Berechnung der Gew.-% von NaCl herangezogen. Sollte beispielsweise auch der Phosphatpuffer in Bestandteil (iv) der Komponente (A) NaCl enthalten, wird auch das NaCl des Phosphatpuffers für die Berechnung der Gew.-% des NaCl in Komponente (iii) herangezogen.

Der Begriff "Phosphatpuffer" wie hierin verwendet beschreibt eine Pufferlösung, die ein oder mehrere Phosphat(e) enthält, insbesondere ausgewählt aus der Gruppe bestehend aus H₃PO₄, H₂PO₄⁻, HPO₄²⁻, PO₄³⁻, sowie deren Salzen, insbesondere Natrium- und Kaliumsalzen, und Mischungen aus den Phosphaten und deren Salzen, insbesondere Natrium- und Kaliumsalzen. Der Begriff "Pufferlösung" beschreibt ein Stoffgemisch, dessen pH-Wert sich bei Zugabe einer Säure oder einer Base deutlich weniger stark ändert, als dies in einem ungepufferten System der Fall gewesen wäre. Vorzugsweise beschreibt der Begriff "Pufferlösung" eine wässrige Lösung.

Vorzugsweise beinhalten die Begriffe "Na₂HPO₄" und "KH₂PO₄", wie hierin verwendet, auch die Hydrate von Na₂HPO₄ bzw. KH₂PO₄. Besonders bevorzugt beinhaltet der Begriff "Na₂HPO₄" die Hydrate Na₂HPO₄*12H₂O und Na₂HPO₄*2H₂O.

Vorzugsweise ist/sind das, ein oder mehrere Phosphat(e) in der Pufferlösung ausgewählt aus der Gruppe bestehend aus Na₂HPO₄, insbesondere Na₂HPO₄*12H₂O, Na₂HPO₄*2H₂O, KH₂PO₄.

Besonders bevorzugt ist/sind das, ein oder mehrere Phosphat(e) in der Pufferlösung Na₂HPO₄ und/oder KH₂PO₄.

Komponente (B) kann zudem NaCl beinhalten.

Es ist bevorzugt, dass in Komponente (A) einer erfindungsgemäßen gelbildenden Mehr-Komponenten-Zusammensetzung
Natriumalginat (Bestandteil (i)) in einem Bereich von 0,5 bis 10 Gew.%, vorzugsweise 0,5 bis 7,5 Gew.-%, besonders bevorzugt 0,5 bis 5 Gew.-%, weiter bevorzugt 0,5 bis 2,5 Gew.-%, zudem bevorzugt 0,5 bis 1,25 Gew.-%; besonders bevorzugt 0,75 bis 1,0 Gew.-%, bezogen auf das Gewicht des Bestandteils (ii), vorliegt,
   und/oder
Na₂HPO₄ (Bestandteil (v)), sofern vorhanden, in einem Bereich von 0,005 bis 0,3 Gew.-%, vorzugsweise 0,01 bis 0,2 Gew.-%, besonders bevorzugt 0,05 bis 0,1 Gew.-%, bezogen auf das Gewicht des Bestandteils (ii), vorliegt,
   und/oder
KH₂PO₄ (Bestandteil (vi)), sofern vorhanden, in einem Bereich von 0,001 bis 0,1 Gew.-%, vorzugsweise 0,005 bis 0,05 Gew.-%, besonders bevorzugt von 0,0075 bis 0,025 Gew.-%, bezogen auf das Gewicht des Bestandteils (ii), vorliegt.

Vorzugsweise sind in Komponente (A) einer erfindungsgemäßen gelbildenden Mehr-Komponenten-Zusammensetzung 0,001 bis 10 Gew.-%, vorzugsweise 0,005 bis 5 Gew.%, besonders bevorzugt 0,01 bis 1 Gew.-%, ganz besonders bevorzugt 0,05 bis 0,5 Gew.% an Phosphatpuffer, wie hierin beschrieben, jeweils bezogen auf das Gewicht des Bestandteils (iii), vorhanden.

Vorzugsweise sind in Komponente (A) einer erfindungsgemäßen gelbildenden Mehr-Komponenten-Zusammensetzung 0,0005 bis 0,1 Gew.-%, besonders bevorzugt 0,00075 bis 0,05 Gew.-%, ganz besonders bevorzugt 0,001 bis 0,01 Gew.-% an einem oder mehreren Phosphat(en), wie hierin beschrieben, jeweils bezogen auf das Gewicht des Bestandteils (iii), vorhanden.

Zusätzlich oder alternativ ist es auch bevorzugt, dass in Komponente (B) einer erfindungsgemäßen gelbildenden Mehr-Komponenten-Zusammensetzung
NaCl (Bestandteil (d)), sofern vorhanden, in einem Bereich von 0,05 bis 1,5 Gew.-%, vorzugsweise 0,1 bis 1,0 Gew.-%, besonders bevorzugt 0,25 bis 0,75 bezogen auf das Gewicht des Bestandteils (b), vorliegt,
   und/oder
CaCl₂ (Bestandteil (a)) in einem Bereich von 0,25 bis 5,0 Gew.-%, vorzugsweise 0,75 bis 2,5 Gew.-%, besonders bevorzugt 1,5 bis 2,0 Gew.-% bezogen auf das Gewicht des Bestandteils (b), vorliegt.

Die Menge der vorhandenen Bestandteile beeinflusst sowohl die Geschwindigkeit der Vernetzungsreaktion, als auch die Stabilität und Flexibilität des vernetzten Gels. Die oben beschriebenen, bevorzugten Mengen gewährleisten, dass möglichst schnell ein stabiles und flexibles Gel gebildet wird, welches gegebenenfalls in einem Stück aus dem Körper entfernt werden kann.

Überraschenderweise wurde zudem gefunden, dass die erfindungsgemäße gelbildende Mehr-Komponenten-Zusammensetzung eine längere Haltbarkeit aufweist. Dies ermöglicht eine Lagerung der einzelnen Komponenten ohne oder mit nur geringen Verlusten der Funktion.

Der Begriff "Sterilisation" beschreibt vorzugsweise ein Verfahren, durch welches ein Material oder ein Gegenstand von Mikroorganismen, vorzugsweise von lebenden Mikroorganismen, und/oder Viren und/oder Prionen und/oder Nukleinsäuren, vorzugsweise Plasmiden, befreit wird oder deren Anzahl reduziert wird und/oder die Mikroorganismen abgetötet werden. Unter Mikroorganismen sind vorzugsweise auch deren Ruhestadien (z.B. Sporen) zu verstehen.

Zusätzlich oder alternativ umfasst der Begriff "Sterilisation" vorzugsweise ein Verfahren, nach dessen Durchführung weder lebende *Escherichia coli* noch lebende Salmonellen vorkommen.

Vorzugsweise beschreibt der Begriff "Sterilisation" ein Verfahren, nach dessen Durchführung die Gesamtzahl aerober Mikroorganismen einen Wert von 10³ KBE/g (KBE: Kolonie-bildende Einheit; englisch: CFU: colony forming unit) und/oder die Gesamtzahl von Hefen und Pilzen einen Wert von 10² KBE/g nicht übersteigt, wobei sich das Gewicht (in Gramm) auf das zu sterilisierende Gut bzw. Material bezieht.

Vorzugsweise beschreibt der Begriff "steril" die Abwesenheit von lebenden Mikroorganismen, wobei der Restgehalt an lebenden Mikroorganismen in einer Einheit des Sterilisierguts höchstens 10⁻⁶ beträgt. Ein Restgehalt von höchstens 10⁻⁶ bedeutet, dass in einer Million gleichbehandelten Einheiten eines Sterilisierguts nur ein lebender Mikroorganismus enthalten ist.

Der Begriff "lebender Mikroorganismus" beschreibt bevorzugt einen vermehrungsfähigen Mikroorganismus.

Vorzugsweise beschreibt der Begriff "steril" zusätzlich oder alternativ die Abwesenheit von vermehrungsfähigen Viren bzw. Virionen, wobei der Restgehalt an vermehrungsfähigen Viren bzw. Virionen in einer Einheit des Sterilisierguts höchstens 10⁻⁶ beträgt. Ein Restgehalt von höchstens 10⁻⁶ bedeutet, dass in einer Million gleichbehandelten Einheiten eines Sterilisierguts nur ein vermehrungsfähiges Virus bzw. Virion enthalten ist.

Vorzugsweise beschreibt der Begriff "steril" zusätzlich oder alternativ die Abwesenheit von Prionen, vorzugsweise funktionsfähigen Prionen, wobei der Restgehalt an Prionen, bzw. funktionsfähigen Prionen, in einer Einheit des Sterilisierguts höchstens 10⁻⁶ beträgt. Ein Restgehalt von höchstens 10⁻⁶ bedeutet, dass in einer Million gleichbehandelten Einheiten eines Sterilisierguts nur ein Prion, bzw. funktionsfähiges Prion, enthalten ist.

Der Begriff "funktionsfähiges Prion" beschreibt bevorzugt ein Prion, das die Konformation eines Proteins derart ändern kann, dass dieses ebenfalls zu einem Prion wird.

Vorzugsweise beschreibt der Begriff "steril" zusätzlich oder alternativ die Abwesenheit von Nukleinsäuren, vorzugsweise funktionsfähigen Nukleinsäuren, wobei der Restgehalt an Nukleinsäuren, bzw. funktionsfähigen Nukleinsäuren, in einer Einheit des Sterilisierguts höchstens 10⁻⁶ beträgt. Ein Restgehalt von höchstens 10⁻⁶ bedeutet, dass in einer Million gleichbehandelten Einheiten eines Sterilisierguts nur ein Nukleinsäuremolekül, bzw. funktionsfähiges Nukleinsäuremolekül, enthalten ist.

Der Begriff "funktionsfähiges Nukleinsäuremolekül" beschreibt bevorzugt ein Nukleinsäuremolekül, das noch transkribiert und/oder translatiert werden kann.

Vorzugsweise beschreibt der Begriff "steril" zusätzlich oder alternativ einen SAL-Wert von mindestens 1 × 10⁻⁶. Der SAL eines Sterilisationsverfahrens wird ausgedrückt als die Wahrscheinlichkeit des Überlebens von Mikroorganismen in einem Produktelement, nachdem dieses das Verfahren durchlaufen hat. Ein SAL von 10⁻⁶ beispielsweise gibt eine Wahrscheinlichkeit von höchstens 1 nicht sterilem Element in 1×10⁶ sterilisierten Elementen des Endprodukts an. Der SAL eines Verfahrens für ein gegebenes Produkt wird durch geeignete Validierungsstudien etabliert (Quelle: Ph. Eur. 10. - 10.0/5.01.01.01.00).

Mögliche Sterilisationsverfahren beinhalten Verfahren mit chemischen Substanzen, mit Hilfe von physikalischen Methoden oder Kombinationen daraus. Insbesondere können solche Verfahren ausgewählt sein aus der Gruppe bestehend aus Dampfsterilisation, Heißluftsterilisation, Bestrahlung, Gassterilisation und Sterilfiltration.

Bei der Dampfsterilisation wird die Luft im Inneren des Sterilisationsgeräts, vorzugsweise eines Autoklaven, durch Wasserdampf ersetzt. Vorzugsweise wird das zu sterilisierende Gut auf eine Temperatur von mindestens 121 °C, bevorzugt in einem Bereich von 121 °C bis 134 °C, gebracht, vorzugsweise bei einem Druck von mindestens 2 bar, bevorzugt in einem Bereich von 2 bis 3 bar. Bevorzugt ist zudem, dass das zu sterilisierende Gut den genannten Bedingungen für mindestens 5 Minuten, bevorzugt mindestens 10 Minuten, besonders bevorzugt mindestens 15 Minuten, vorzugsweise einer Zeit im Bereich von 5 bis 25 Minuten ausgesetzt wird.

Bevorzugt wird das zu sterilisierende Gut für mindestens 20 Minuten einer Temperatur von mindestens 121 °C bei mindestens 2 bar Druck im Wasserdampf ausgesetzt.

Bevorzugt wird das zu sterilisierende Gut für mindestens 5 Minuten einer Temperatur von mindestens 134 °C bei mindestens 3 bar Druck im Wasserdampf ausgesetzt.

Vorzugsweise weist das Sterilisationsverfahren einen F₀-Wert von mindestens 8 auf.

Bei der Heißluftsterilisation wird das zu sterilisierende Gut vorzugsweise einer Temperatur von mindestens 160 °C, bevorzugt mindestens 170 °C, besonders bevorzugt mindestens 180 °C, insbesondere bevorzugt mindestens 220 °C ausgesetzt. Vorzugsweise wird das zu sterilisierende Gut der Temperatur für mindestens 30 Minuten, bevorzugt mindestens 60 Minuten, besonders bevorzugt mindestens 120 Minuten ausgesetzt.

Bei der Bestrahlung wird das zu sterilisierende Gut ionisierender Strahlung ausgesetzt. Die ionisierende Strahlung ist dabei vorzugsweise ausgewählt aus UV-Strahlung, Röntgenstrahlung, Beta-Strahlung und Gamma-Strahlung. Vorzugsweise beträgt die durch ionisierende Strahlung verursachte Energiedosis mindestens 25 kGy.

Bei der Gassterilisation wird das zu sterilisierende Gut einem Gas ausgesetzt, das z.B. Mikroorganismen abtötet. Vorzugsweise ist das Gas Ethylenoxid.

Bei der Sterilfiltration wird das zu sterilisierende Gut filtriert. Vorzugsweise wird ein Filter mit einer Porengröße von maximal 0,25 µm, bevorzugt maximal 0,22 µm, besonders bevorzugt maximal 0,2 µm, insbesondere bevorzugt maximal 0,1 µm eingesetzt.

Vorzugsweise wird bei der Sterilfiltration nach der Filtration ein Bubble-Point-Test zur Qualitätsprüfung des Filters durchgeführt.

Vorteilhafterweise erfolgt die Sterilisation von Medizinprodukten in der Endverpackung, da dieses Vorgehen einen einfacheren Nachweis für ein steriles, angebotenes Produkt ermöglicht und eine einfachere Aufrechterhaltung des (gesamten) Herstellungsverfahrens bietet.

Überraschenderweise wurde auch gefunden, dass die erfindungsgemäße gelbildende Mehr-Komponenten-Zusammensetzung auch in Anwesenheit wässriger Lösungen, z.B. einer physiologischen Kochsalzlösung, ein Gel bildet. Bei bereits bekannten Zusammensetzungen oder Klebstoffen ist dies typischerweise nicht der Fall bzw. diese können nicht in Anwesenheit wässriger Lösungen ein geeignetes Gel bilden, das eine ausreichende Festigkeit aufweist.

Weiter vorzugsweise umfasst bzw. umfassen Komponente (A) und/oder Komponente (B) einer erfindungsgemäßen gelbildenden Mehr-Komponenten-Zusammensetzung mindestens einen Farbstoff. Dabei ist es besonders bevorzugt, dass der Farbstoff physiologisch unbedenklich ist und somit in der Anwendung an / in einem Patienten unproblematisch eingesetzt werden kann.

Bei der Entfernung von unerwünschten Partikeln aus einem Patienten hat sich gezeigt, dass ein gefärbtes Gel einige Vorteile mit sich bringt. Die genaue Menge und der genaue Ort der injizierten Komponente(n) lässt sich so präzise kontrollieren. Auch kann ermittelt werden, ob die Gelbildung bereits abgeschlossen ist und das gebildete Gel lässt sich endoskopisch leicht wiederfinden. Zudem kann festgestellt werden, ob die jeweiligen Komponenten durch die Verwendung einer Spüllösung oder einer zu starken Dosierung verwirbelt werden, wodurch die Komponenten gegebenenfalls so stark verdünnt würden, dass eine ordnungsgemäße Funktion nicht gewährleistet werden kann.

Um solche Vorteile zu nutzen, beschreibt Cloutier J, Cordeiro ER, Kamphuis GM, Villa L, Letendre J, de la Rosette JJ, Traxer O (2014) "The glueclot technique: a new technique description for small calyceal stone fragments removal" Urolithiasis 42 (5):441-444, autologes Blut zu verwenden. Die Bildung eines Blutklumpens aus geronnenem Blut dauert jedoch deutlich länger als die Bildung eines gefestigten Gels aus den Komponenten wie hierin beschrieben. Auch erschwert der geringe Farbkontrast zwischen Blut und dem Gewebe des Patienten die Anwendung. Weiterhin wünscht der behandelnde Arzt die eingebetteten Steinfragmente zu sehen, als Erfolgskontrolle des Eingriffes. Im Blutklumpen ist dieses jedoch nicht machbar wegen der starken Eigenfarbe des Blutes. Eine Verdünnung des Blutes würde aber die Bildung eines festen Blutklumpens verhindern bzw. dessen Festigkeit stark reduzieren. Zusätzlich kann das Eigenblut nicht mit Hilfe eines feinen Katheters gezielt im Bereich der Steinfragmente z.B. in einem distalen Nierenkelch appliziert werden (Blut hat eine zu hohe Viskosität hierfür).

Zudem ist es bevorzugt, dass der bzw. mindestens einer des mindestens einen Farbstoffes einen hohen Kontrast zum Gewebe des Patienten, in welchem die Mehr-Komponenten-Zusammensetzung eingesetzt wird, hat. So ist es bevorzugt, dass der bzw. mindestens einer des mindestens einen Farbstoffes nicht für eine rote oder gelb-orangene Färbung sorgt.

Weiter bevorzugt ist, dass der bzw. mindestens einer des mindestens einen Farbstoffes nicht für eine schwarze oder weiße Färbung sorgt. Dies ermöglicht eine noch bessere Erfolgskontrolle, ob die zu entfernenden Partikel bereits durch das Gel umschlossen sind bzw. welche der Partikel bereits umschlossen sind und wo noch ein Gel zur Entfernung der Partikel benötigt wird.

Besonders vorteilhaft ist es auch, wenn der Farbstoff nicht aus dem gefestigten Gel und/oder aus der bzw. einer Komponente enthaltend den Farbstoff austritt. In diesem Fall wird kein umliegendes Gewebe des Patienten angefärbt und der Farbstoff kann als Indikator für das Gel bzw. die bzw. eine Komponente enthaltend den Farbstoff dienen. Eine Erfolgskontrolle, ob das gefestigte Gel wieder vollständig aus dem Patienten entfernt wurde, ist daher möglich. Zudem lässt sich eine angefärbte Komponente besser dosieren in Hinblick auf die Menge als auch auf den tatsächlichen Ort der Aufbringung. Auch wird die Verwechslung von Gel und Gewebe des Patienten minimiert und bei der anschließenden Entfernung des Gels nur das Gel selbst samt der davon umschlossenen Partikel und nicht (körpereigenes) Gewebe des Patienten, das nicht entfernt werden soll, entfernt. Versehentliche Verletzungen des umliegenden Patientengewebes werden somit minimiert.

Weiter ist es bevorzugt, dass Komponente (A) und Komponente (B) mindestens einen Farbstoff umfassen und wobei der/die Farbstoff(e) in Komponente (A) unterschiedlich ist/sind von dem/den Farbstoffen) in Komponente (B). Dies hat sich insbesondere dadurch als vorteilhaft erwiesen, da so farblich zwischen den Komponenten (A) und (B) unterschieden werden kann. Auch ist so vorzugsweise und vorteilhafterweise möglich, festzustellen, ob sich die Komponenten (A) und (B) bereits gemischt haben bzw. vermischt wurden, da durch eine Mischung der beiden Komponenten vorzugsweise eine Farbe entsteht, die sich von der Farbe der gefärbten Komponenten (A) und/oder (B) unterscheiden.

Der Ausdruck "wobei der/die Farbstoff(e) in Komponente (A) unterschiedlich ist/sind von dem/den Farbstoffen) in Komponente (B)" ist so zu verstehen, dass im Falle von mehreren Farbstoffen in einer oder beiden der Komponenten (A) und (B) die Kombination an Farbstoffen der einen Komponente nicht mit der Kombination an bzw. dem Farbstoffen) übereinstimmt. Folglich können beide Komponenten den gleichen Farbstoff enthalten, solange mindestens eine der Komponenten noch einen weiteren, anderen Farbstoff enthält.

Es ist insbesondere bevorzugt, dass eine der beiden Komponenten (A) und (B) Dextranblau umfasst. Es wurde überraschenderweise gefunden, dass insbesondere Dextranblau zusätzlich den Vorteil mit sich bringt, dass dieser Farbstoff nicht aus dem Gel herausdiffundiert und daher auch nicht das umliegende Gewebe anfärbt, was bei der Entfernung des Gels mitsamt umschlossenen Partikeln, ohne aber einer Verletzung des umgebenden Gewebes, sowie bei der Kontrolle, ob das Gel vollständig entfernt wurde, besonders vorteilhaft ist.

Besonders bevorzugt umfasst die eine der beiden Komponenten (A) und (B) Dextranblau und die andere der beiden Komponenten (A) und (B) Riboflavin.

Vorzugsweise liegt in einer der beiden Komponenten (A) und (B) Dextranblau in einem Bereich von 0,01 bis 1,0 Gew.-%, vorzugsweise 0,05 bis 0,75 Gew.-%, besonders bevorzugt 0,1 bis 0,5 Gew.-%, bezogen auf das Gewicht des Bestandteils (ii) bzw. des Bestandteils (b), vor.

Zusätzlich oder alternativ liegt in einer der beiden Komponenten (A) und (B) Riboflavin in einem Bereich von 0,0001 bis 0,05 Gew.-%, vorzugsweise 0,0005 bis 0,01 Gew.-%, besonders bevorzugt 0,001 bis 0,005 Gew.-%, bezogen auf das Gewicht des Bestandteils (ii) bzw. des Bestandteils (b), vor.

Der Ausdruck "bezogen auf das Gewicht des Bestandteils (ii) bzw. des Bestandteils (b)" ist so zu verstehen, dass sich die Angabe "Gew.-%" auf das Gewicht des Bestandteils (ii) bezieht, sofern Dextranblau bzw. Riboflavin in Komponente (A) vorhanden ist, wohingegen sich die Angabe "Gew.-%" auf das Gewicht des Bestandteils (b) bezieht, sofern Dextranblau bzw. Riboflavin in Komponente (B) vorhanden ist.

Dies ist besonders vorteilhaft, da so eine Färbung des Gels erzielt wird, die einen hohen Kontrast zum umliegenden Gewebe des Patienten hat. Gleichzeitig ermöglicht Dextranblau bzw. Riboflavin in dieser Konzentration eine besonders gute Erfolgskontrolle, wie oben beschrieben.

Besonders bevorzugt weist die Komponente (A) und/oder die Komponente (B) und/oder eine weitere, gegebenenfalls in der Zusammensetzung enthaltene Komponente (C) einen neutralen pH-Wert auf, vorzugsweise einen pH-Wert im Bereich von 6,5 bis 8,0, besonders bevorzugt einen pH-Wert im Bereich von 7,0 bis 7,5. Ein neutraler pH-Wert hat sich insbesondere als unschädlich für das Körpergewebe erwiesen. Zudem werden die Komponenten bei einer Sterilisation, z.B. Dampfsterilisation, Autoklavieren, nicht oder fast nicht degradiert oder beschädigt.

Eine erfindungsgemäße Zusammensetzung kann zusätzlich weitere Komponenten enthalten. Beispielsweise können den Komponenten (A) und/oder (B) und/oder einer oder mehreren weiteren Komponente einer erfindungsgemäßen Zusammensetzung Substanzen, die die Gelbildung und/oder die Einbindung der Partikel, fördern, zugegeben werden. Solche Substanzen können z.B. Quervernetzer zur Erhöhung der Stabilität des Gels sein.

Erfindungsgemäß ist es daher bevorzugt, dass die Komponente (A), die Komponente (B) und/oder eine weitere, gegebenenfalls in der Zusammensetzung enthaltenen Komponente (C) eine oder mehrere Substanzen zum Verbessern der Vernetzung und/oder der Stabilität des Natriumalginats enthält, insbesondere Quervernetzer, vorzugsweise wobei die, eine mehrere oder alle Substanz(en) ausgewählt ist/sind aus der Gruppe bestehend aus Aminosäuren, (Bio)Polymeren, Zuckerpolymeren, synthetischen Di- oder Multimeren, Zucker-Präpolymeren und synthetischen Präpolymeren.

Eine erfindungsgemäße Zusammensetzung kann zusätzlich oder alternativ weitere Komponenten enthalten. Beispielsweise können den Komponenten (A) und/oder (B) und/oder einer oder mehreren weiteren Komponenten einer erfindungsgemäßen Zusammensetzung Substanzen, die die Gelbildung und/oder die Einbindung der Partikel sowie die Dichte, fördern, zugegeben werden.

Solche Substanzen können z.B. Substanzen zur Erhöhung der Dichte der Gelkomponenten, insbesondere des vernetzbaren Polymers, vorzugsweise des Natriumalginats, sein. Solche Substanzen können ausgewählt sein aus der Gruppe bestehend aus (Bio)Polymeren, Zuckerpolymeren, synthetische Polymere, Zucker-Präpolymere, Zuckermonomere, Zuckerdimere wie Saccharose oder Glucose, synthetische Präpolymere, hydrophile Copolymere, Epichlorhydrin (Ficoll ^{®}) oder Glycerin.

Erfindungsgemäß ist es daher bevorzugt, dass die Komponente (A), die Komponente (B) und/oder eine weitere, gegebenenfalls in der Zusammensetzung enthaltenen Komponente (C) eine oder mehrere Substanzen zum Erhöhen der Dichte des vernetzbaren Polymers aus Bestandteil (i) der Komponente (A), vorzugsweise des Natriumalginats, enthält, vorzugsweise wobei die, eine mehrere oder alle Substanz(en) ausgewählt ist/sind aus der Gruppe bestehend aus (Bio)Polymeren, Zuckerpolymeren, synthetische Polymere, Zucker-Präpolymere, Zuckermonomere, Zuckerdimere wie Saccharose oder Glucose, synthetische Präpolymere, hydrophile Copolymere, Epichlorhydrin (Ficoll ^{®}) oder Glycerin.

Vorzugsweise enthält Komponente (A), Komponente (B) und/oder eine weitere, gegebenenfalls in der Zusammensetzung enthaltene Komponente (C) eine oder mehrere Substanz(en) zum Erhöhen der Dichte des vernetzbaren Polymers aus Bestandteil (i) der Komponente (A), vorzugsweise des Natriumalginats, wie hierin beschrieben, in einer Menge von 1 bis 30 Gew.-%, vorzugsweise von 3 bis 20 Gew.-%, besonders bevorzugt von 5 bis 10 Gew.-%, basierend auf dem Gesamtgewicht der Komponente (A) bzw. der Komponente (B) bzw. der weiteren, gegebenenfalls in der Zusammensetzung enthaltenen Komponente (C). Sofern mehrere der Komponenten in der Zusammensetzung eine oder mehrere solcher Substanzen enthalten, wird die Menge für jede Komponente vorzugsweise einzeln berechnet.

Sofern die bzw. eine der Substanz(en) zur Erhöhung der Dichte ein vernetzbares Polymer gemäß Bestandteil (i) der Komponente (A) ist, wird zur Berechnung des Gewichts des Bestandteils (i) der Komponente (A) oder zur Berechnung von Angaben, die auf dem Gewicht des Bestandteils basieren, das vernetzbare Polymer als Teil des Bestandteils (i) der Komponente (A) berücksichtigt.

Vorzugsweise beschreibt der Begriff "Substanz zur Erhöhung der Dichte der Gelkomponenten, insbesondere des vernetzbaren Polymers, vorzugsweise des Natriumalginats" nicht das eingesetzte vernetzbare Polymer des Bestandteils (i) der Komponente (A) der erfindungsgemäßen Zusammensetzung.

Weiter betrifft die vorliegende Erfindung eine erfindungsgemäße Zusammensetzung, zur Anwendung in einem Verfahren zum Entfernen von unerwünschten Partikeln aus einem Patienten.

Das vorstehend genannte Entfernen von unerwünschten Partikeln stell dabei ein invasives, chirurgisches Verfahren dar. Eine rein kosmetische Anwendung ist hiervon nicht betroffen. Das Entfernen von unerwünschten Partikeln wird gezielt durchgeführt, die unerwünschten Partikel werden dabei bewusst aus dem Körper entfernt.

Unerwünschte Partikel können Partikel jeglicher Art sein, die aus medizinischer Sicht aus dem Körper des Patienten entfernt werden müssen oder können.

Vorzugsweise sind die Partikel Ablagerungen, Ausfallprodukte, Fremdkörper und/oder Fragmente davon und/oder Splitter körpereigener Strukturen. Ablagerungen können insbesondere Harnsteine oder Nierensteine sein. Unter Ausfallprodukten sind Partikel wie beispielsweise Gallensteine als Ausfallprodukt der Gallenflüssigkeit oder der Bauspeicherdrüse oder Speicheldrüsen zu verstehen. Fremdkörper können beispielsweise mineralische Fragmente, Metall-, Kunststoff- oder Holzsplitter sein, die beispielsweise bei Verletzungen wie Schürfwunden in den Körper gelangen. Der Begriff "Fragmente davon" bezieht sich auf die vorgenannten Partikelformen und verdeutlicht, dass diese vor oder während der Entfernung in kleinere Teile bzw. Bruchstücke zerkleinert werden können. Auch ist hierbei möglich, dass durch die Anwendung nur Teile (Fragmente) entfernt werden, während die restlichen Fragmente in einem weiteren Schritt oder einem weiteren Verfahren entfernt werden. Splitter körpereigener Strukturen können beispielsweise bei Operationen, wie dem Einbringen oder Entfernen von Implantaten, entstehen und können daher beispielsweise Knochensplitter oder Fräsreste sein. Hierunter fallen bevorzugt aber auch Splitter anderer Strukturen wie z.B. Knorpelgewebe oder Tumorfragmente, die beispielsweise bei einem vorhergehenden oder gleichzeitig durchgeführten chirurgischen Eingriff entstanden sind. Der Begriff Splitter umfasst dabei Strukturen jeglicher Form und Größe.

Bevorzugt erfolgt das Entfernen der unerwünschten Partikel als ein Verfahren enthaltend die folgenden Schritte:
(i) Bereitstellen der sterilisierten Komponente (A) und (B) und weitere Komponente(n) (sofern vorhanden),
(ii) Einbringen der Komponente (A) und (B) und weitere Komponente(n) (sofern vorhanden) in den Körper des Patienten in einen Bereich der zu entfernende Partikel enthält,
   unter Bedingungen, die bei Kontakt der Komponente (A) mit der Komponente (B) eine Vernetzung des Natriumalginats ermöglichen, so dass ein vernetztes Gel entsteht, das die zu entfernenden Partikel teilweise oder vollständig umschließt,
(iii) Entfernen des vernetzten Gels samt der davon umschlossenen Partikel aus dem Bereich des Körpers des Patienten.

Die Komponenten (A), (B) und gegebenenfalls weitere Komponenten (sofern vorhanden) können in Schritt (ii) gleichzeitig oder nacheinander eingebracht werden. Auch können Komponenten vor Einbringen in den Körper gemischt werden. Einzelne Komponenten können auch vor Einbringen in den Körper gemischt werden, während andere Komponenten erst im Körper mit einem oder mehreren anderen Komponenten in Kontakt gebracht werden. Die Komponenten (A), (B) und gegebenenfalls weitere Komponenten (sofern vorhanden) können auch gleichzeitig, aber separat, d.h. in voneinander getrennter Form, in den Körper eingebracht werden.

Vorzugsweise umfasst das Verfahren den folgenden weiteren Schritt, der zeitlich vor Schritt (ii) erfolgt:
Fragmentieren eines oder mehrerer Partikel in dem Bereich des Körpers des Patienten, so dass zwei oder mehrere, vorzugsweise eine Vielzahl von Fragmenten des/der Partikel(s) entsteht bzw. entstehen.

Wird dieser Schritt ausgeführt, können die Partikel bereits als Fragmente vorliegen, die durch diesen Schritt dann wiederum in Fragmente ihrerseits zerteilt werden.

Bevorzugt umfasst der Begriff "Vielzahl von Fragmenten" mindestens 5, vorzugsweise mindestens 10, besonders bevorzugt mindestens 15, ganz besonders bevorzugt mindestens 20 Fragmente. Liegt ein Partikel, der in diesem Schritt fragmentiert wird, bereits als Fragment vor, so bezieht sich der Begriff "Vielzahl von Fragmenten des/der Partikel(s)" auf den bereits fragmentierten Partikel und die Fragmente die aus diesem entstehen.

Zudem betrifft die vorliegende Erfindung ein Verfahren zur Herstellung einer erfindungsgemäßen Zusammensetzung, umfassend oder bestehend aus den Schritten:
(i) Bereitstellen der Komponente (A), wie hierin beschrieben,
(ii) Bereitstellen der Komponente (B), wie hierin beschrieben,
(iii) optional: Bereitstellen einer Komponente (C), wie hierin beschrieben,
(iv) Sterilisieren der in den vorangehenden Schritten bereitgestellten Komponente (A) und optional Komponente (B) und/oder Komponente (C) (sofern vorhanden).

Was oben zu den Eigenschaften und Vorteilen des Gels, der gelbildenden Mehr-Komponenten-Zusammensetzung, der Komponenten oder der einzelnen Bestandteile der gelbildenden Mehr-Komponenten-Zusammensetzung gesagt wurde, gilt auch für die Komponenten oder die einzelnen Bestandteile in dem Herstellungsverfahren entsprechend. So können beispielsweise, wie oben bereits beschrieben, die Komponenten (A) und/oder (B) mindestens einen Farbstoff, wie oben beschrieben, enthalten. Besonders bevorzugt kann Komponente (A) Na₂HPO₄ und/oder KH₂PO₄ beinhalten, wie oben beschrieben. Alternativ oder zusätzlich kann Komponente (B) NaCl beinhalten, wie oben beschrieben.

Vorzugsweise ist die Sterilisation in Schritt ii) des erfindungsgemäßen Verfahrens ausgewählt aus der Gruppe bestehend aus Dampfsterilisation, Heißluftsterilisation, Bestrahlung, Gassterilisation und Sterilfiltration.

Gegenüber der Heißluftsterilisation bietet die Dampfsterilisation den Vorteil, dass diese auch bei Biomaterialien und vielen Kunststoffen eingesetzt werden kann. Denn durch die Dampfsterilisation werden diese Materialien nicht (bzw. nur in geringerem Maße) zerstört.

Gegenüber der Bestrahlung bietet die Dampfsterilisation den Vorteil, dass diese Biopolymere - im Vergleich zur hochenergetischen Strahlung, wie z.B. Gamma- oder Betastrahlung - nicht (bzw. nur in geringerem Maße) zerstört. Für das Abtöten von Mikroorganismen ist es zwar gewünscht, dass die Biomoleküle, welche die Basis der lebenden Organismen bilden, zerstört werden. Im Fall von Medizinprodukten, welche funktionelle Biomoleküle in Form von Biopolymere enthalten, führt eine hochenergetische Bestrahlung aber auch zum drastischen Funktionsverlust durch Kettenfragmentierung.

Gegenüber der Gassterilisation bietet die Dampfsterilisation den Vorteil, dass auch Medizinprodukte in der Endverpackung vorteilhaft sterilisiert werden können. Bei beispielsweise der Gassterilisation mit Ethylenoxid würde dieses nicht oder nur kaum mit der zu sterilisierenden Substanz in Kontakt gelangen, wenn diese in der Endverpackung sterilisiert werden soll.

Auch gegenüber der Sterilfiltration bietet die Dampfsterilisation den Vorteil, dass Medizinprodukte in der Endverpackung vorteilhaft sterilisiert werden können. Die Sterilfiltration ist nicht der letzte Schritt in der Produktion und Verpackung eines Medizinprodukts und stellt somit kein terminales Verfahren dar.

Vorzugsweise enthält Schritt (iv) des erfindungsgemäßen Verfahrens einen Schritt der Dampfsterilisation oder besteht daraus,
vorzugsweise wobei die Temperatur bei der Dampfsterilisation mindestens 121 °C beträgt, besonders vorzugsweise in einem Bereich von 121 °C bis 134 °C liegt,
vorzugsweise wobei der Druck bei der Dampfsterilisation mindestens 2 bar beträgt, besonders vorzugsweise in einem Bereich von 2 bis 3 bar liegt,
und vorzugsweise wobei die Dauer der Dampfsterilisation mindestens 5 Minuten beträgt, besonders vorzugsweise in einem Bereich von 5 bis 25 Minuten liegt.

Besonders bevorzugt ist die Sterilisation in Schritt iv) des erfindungsgemäßen Verfahrens eine Dampfsterilisation, vorzugsweise wie hierin beschrieben, wobei die bereitgestellte(n) Komponente(n) für mindestens 15 Minuten, vorzugsweise mindestens 20 Minuten, einer Temperatur von mindestens 121 °C bei mindestens 2 bar Druck im Wasserdampf ausgesetzt wird.

Ganz besonders bevorzugt ist ein Verfahren, bei welchem eine Komponente (A), wie hierin beschrieben, bereitgestellt wird, wobei die Komponente (A), vorzugsweise der Phosphatpuffer der Komponente (A), Na₂HPO₄ oder KH₂PO₄ umfasst oder wobei die Komponente (A), vorzugsweise der Phosphatpuffer der Komponente (A), Na₂HPO₄ und KH₂PO₄ umfasst.

Weiter bevorzugt ist ein Verfahren, bei welchem eine Komponente (A), wie hierin beschrieben, bereitgestellt wird, wobei die Komponente (A), vorzugsweise der Phosphatpuffer der Komponente (A), Na₂HPO₄ oder KH₂PO₄ umfasst oder wobei die Komponente (A), vorzugsweise der Phosphatpuffer der Komponente (A), Na₂HPO₄ und KH₂PO₄ umfasst und wobei die Sterilisation in Schritt ii) des erfindungsgemäßen Verfahrens eine Dampfsterilisation ist, vorzugsweise wie hierin beschrieben, vorzugsweise wobei die Komponente (A) und optional Komponente (B) und/oder Komponente (C) (sofern vorhanden) für mindestens 15 Minuten, vorzugsweise mindestens 20 Minuten, einer Temperatur von mindestens 121 °C bei mindestens 2 bar Druck im Wasserdampf ausgesetzt wird.

Vorzugsweise weist die Sterilisation in Schritt iv) des erfindungsgemäßen Verfahrens, insbesondere den als bevorzugt beschriebenen Verfahren, einen F₀-Wert von mindestens 8. bevorzugt mindestens 10, besonders bevorzugt mindestens 15 auf.

Insbesondere bevorzugt ist ein Verfahren, bei welchem eine Komponente (A), wie hierin beschrieben, bereitgestellt wird, wobei die Komponente (A), vorzugsweise der Phosphatpuffer der Komponente (A), Na₂HPO₄ oder KH₂PO₄ umfasst oder wobei die Komponente (A), vorzugsweise der Phosphatpuffer der Komponente (A), Na₂HPO₄ und KH₂PO₄ umfasst und wobei die Sterilisation in Schritt iv) des erfindungsgemäßen Verfahrens eine Dampfsterilisation ist, vorzugsweise wobei die Komponente (A) und optional Komponente (B) und/oder Komponente (C) (sofern vorhanden) für mindestens 15 Minuten, vorzugsweise mindestens 20 Minuten, einer Temperatur von mindestens 121 °C bei mindestens 2 bar Druck im Wasserdampf ausgesetzt wird, wobei die Sterilisation in Schritt ii) einen F₀-Wert von mindestens 8. bevorzugt mindestens 10, besonders bevorzugt mindestens 15 aufweist.

Weiter betrifft die vorliegende Erfindung eine erfindungsgemäße Zusammensetzung, hergestellt oder herstellbar durch ein erfindungsgemäßes Verfahren.

Im Folgenden wird die vorliegende Erfindung anhand ausgewählter Beispiele näher erläutert.

### Beispiele

### Beispiel 1: Herstellung von Komponenten (A), (B) und (C)

Zur Herstellung einer beispielhaften Komponente (A) werden 5 g Dextranblau und 4 g Natriumalginat in 1 L Wasser gelöst.

Das Natriumalginat hat eine molare Masse von 200.000 g/mol und einen G-Anteil von 55 %.

Zur Herstellung einer beispielhaften Komponente (B) werden 10 g Calciumchlorid-Dihydrat in 1 L Wasser gelöst.

Zur Herstellung einer beispielhaften Komponente (C) wird eine 4 bis 40 mM Eisen (0,35 bis 3,5 g pro Liter) enthaltende Partikel-Suspension in Wasser oder physiologischem Puffer zubereitet (M. Geppert et al., Nanotechnology 22 (2011) 145101). Diese Lösung wird zu 1% bis 50% zu A oder B gegeben.

### Beispiel 2: Anwendung eines erfindungsgemäßen gelbildenden Systems zur Entfernung von Harnsteinen

Ein Zugang zum Harntraktlumen (z.B. zum Nierenbeckenkelchsystem) wird entweder ureterorenoskopisch (durch Harnröhre, Blase und Harnleiter) oder perkutan (durch Hautpunktion an der Flanke) geschaffen. Eine spezielle Schleuse (ggf. ein Polymerrohr mit Metallanteilen) mit einem Innendurchmesser von 3 bis 9 mm wird darin platziert. Über den vorgelegten Zugangsschaft wird ein Endoskop ins Harntraktlumen (z.B. ins Nierenbeckenkelchsystem) eingebracht, das Operationsgebiet inspiziert und der Harnstein bzw. die Harnsteine visualisiert. Der Harnstein bzw. die Harnsteine werden z. B. mittels eines Holmium-Lasers zertrümmert. Die großen und mittelgroßen Fragmente werden mit einem Steinfanginstrument entfernt. Anschließend wird ein Katheter über das Endoskopgerät (durch den Zugang) eingeführt und bis zu 3 mL, insbesondere 300 bis 500 µL einer Komponente (A) gemäß Beispiel 1 in den Bereich des Harntrakts (z.B. ins Nierenbeckenkelchsystem) so appliziert, dass A die Steine bzw. die Fragmente des bzw. der zertrümmerten Harnsteine(s) umschließt bzw. einbettet. Danach werden, ebenfalls über den im Endoskop befindlichen Katheter, bis zu 9 mL einer Komponente (B) gemäß Beispiel 1 in die Nähe von B appliziert. Eine aktive Vermischung von A mit B ist nicht notwendig. Innerhalb weniger Sekunden bis einer Minute kommt es zu einer Gelbildung.

Der Katheter kann mit 0,9 % NaCl-Lösung gespült werden zwischen der Applikation von A und B. Dann wird ein Greifinstrument über das Operationsendoskop über die Zugangsschleuse eingeführt. Mit dem Greifinstrument wird das gefestigte Gel in einem Stück oder in mehreren Teilen gefasst und durch Extraktion aus dem Körper entfernt.

### Beispiel 3: Vergleich verschiedener qelbildender Zusammensetzungen vor und nach der Sterilisation

Die nachfolgenden Formulierungen, enthaltend Natriumalginat, wurden bereitgestellt:

| | **1 (Vergleich)** | **2 (Vergleich)** | **3 (erfindungsgemäß)** |
|---|---|---|---|
| **Natriumalginat** | 0,8 g | 0,8 g | 0,8 g |
| **Wasser** | 98,3 g | 98,9 g | 98,0 g |
| **NaCl** | 0,9 g | - | 0,9 g |
| **PBS** | - | 0,3 g (enthaltend 0,01 g NaCl) | 0,3 g (enthaltend 0,01 g NaCl) |
| **pH** | 7,4 | 7,4 | 7,4 |
| **Total** | 100 g | 100 g | 100 g |

Die Formulierungen wurden jeweils per Dampfsterilisation für 20 Minuten bei 121 °C sterilisiert.

Wie oben beschrieben, führt auch die Dampfsterilisation zu Brüchen in den Natriumalginatketten, so dass längere Molekülketten sich in kürzere Ketten umwandeln. Diese Zerkleinerung vom Molekülketten kann unter anderem durch die Messung der Viskosität analysiert werden. Für die Viskositätsmessung ist in der Literatur beschrieben, dass mit sich verkürzenden Kettenlängen auch der Wert der Viskosität (meist in der Einheit mPa*s angegeben) geringer wird. Die Verringerung der Molekülkettenlänge geht meist auch mit einem Verlust der Funktionalität einher.

Daher wurde die Viskosität der Formulierungen vor und nach der Dampfsterilisation mit Hilfe eines Viskosimeters bei 25 °C gemessen.

Hierfür wurde ein DV2T Viskosimeter der Firma Brookfield-AMETEK mit einer CPA-41Z Spindel verwendet, wobei die Temperatur mit Hilfe eines TC-550 Badthermostates der Firma Brookfield-AMETEK konstant gehalten wurde. Ebenso wurde die Gelbildungsfähigkeit gemessen. Hierfür wurden die jeweiligen Komponenten in einer Schale zusammengegeben im Bereich der Kontaktflächen der Flüssigkeiten mit Hilfe eines Greifers (zum Beispiel einer Pinzette) ein Hydrogelfaden herausgezogen, sofern möglich.

Die folgenden Ergebnisse wurden für die einzelnen Formulierungen erhalten:

| | 1 (Vergleich) | 2 (Vergleich) | 3 (erfindungsgemäß) |
|---|---|---|---|
| Viskosität vor Sterilisation [mPa*s] | 157 | 123 | 135 |
| Viskosität nach Sterilisation [mPa*s] | 5 | 3 | 39 |
| Viskosität nach Sterilisation [%] | 3 | 2 | 29 |
| Gelbildung vor Sterilisation | ja | ja | ja |
| Gelbildung nach Sterilisation | nein | nein | ja |

Die Viskosität nach Sterilisation [%] berechnet sich als Quotient aus der Viskosität nach Sterilisation [mPa*s] und der Viskosität vor Sterilisation [mPa*s].

Die Viskosität der erfindungsgemäßen Formulierung 3 war nach der Sterilisation um den Faktor 7,8 gegenüber Formulierung 1 erhöht. Im Vergleich zu Formulierung 2 war die Viskosität nach der Sterilisation sogar um den Faktor 13 erhöht. Eine Gelbildung nach der Sterilisation war nur bei der erfindungsgemäßen Formulierung 3 möglich.

## Patentansprüche

1. Sterilisierte, gelbildende Mehr-Komponenten-Zusammensetzung, vorzugsweise Zwei-Komponenten-Zusammensetzung, zur Bildung eines Gels,
die Zusammensetzung bestehend aus oder umfassend
Komponente (A), enthaltend
(i) ein oder mehrere vernetzbare, vorzugsweise kationisch vernetzbare, Polymer(e), wobei das oder ein Polymer Natriumalginat ist,
(ii) Wasser,
(iii) NaCl in einem Bereich von 0,3 bis 1,2 Gew.-%, basierend auf dem Gesamtgewicht der Komponente (A),
(iv) einen oder mehrere Phosphatpuffer und
und
Komponente (B), enthaltend
(a) ein oder mehrere Vernetzungsmittel zum Vernetzen des bzw. der vernetzbaren Polymer(e) in Bestandteil (i) der Komponente (A), vorzugsweise wobei das oder ein Vernetzungsmittel CaCl₂ ist, und
(b) Wasser,

2. Sterilisierte, gelbildende Zusammensetzung nach Anspruch 1, wobei der Phosphatpuffer in Komponente (A) ein oder mehrere Phosphat(e) enthält, die ausgewählt sind aus der Gruppe bestehend aus H₃PO₄, H₂PO₄⁻, HPO₄²⁻, PO₄³⁻, sowie deren Salzen, vorzugsweise Natrium- und Kaliumsalzen, und Mischungen aus den Phosphaten und deren Salzen, vorzugsweise Natrium- und Kaliumsalzen.

3. Sterilisierte, gelbildende Zusammensetzung nach einem der vorangehenden Ansprüche,
wobei in Komponente (A)
Natriumalginat (Bestandteil (i)) in einem Bereich von 0,5 bis 10 Gew.-%, vorzugsweise 0,5 bis 7,5 Gew.-%, besonders bevorzugt 0,5 bis 5 Gew.-%, weiter bevorzugt 0,5 bis 2,5 Gew.-%, zudem bevorzugt 0,5 bis 1,25 Gew.-%; besonders bevorzugt 0,75 bis 1,0 Gew.-%, bezogen auf das Gewicht des Bestandteils (ii), vorliegt.
und/oder
wobei in Komponente (B)
CaCl₂ (Bestandteil (a)) in einem Bereich von 0,25 bis 5,0 Gew.-%, vorzugsweise 0,75 bis 2,5 Gew.-%, besonders bevorzugt 1,5 bis 2,0 Gew.-% bezogen auf das Gewicht des Bestandteils (b), vorliegt.

4. Sterilisierte, gelbildende Zusammensetzung nach einem der vorangehenden Ansprüche, wobei Komponente (A) und/oder Komponente (B) mindestens einen Farbstoff umfasst/umfassen.

5. Sterilisierte, gelbildende Zusammensetzung nach Anspruch 4, wobei Komponente (A) und Komponente (B) mindestens einen Farbstoff umfassen und wobei der/die Farbstoff(e) in Komponente (A) unterschiedlich ist/sind von dem/den Farbstoffen) in Komponente (B).

6. Sterilisierte, gelbildende Zusammensetzung nach Anspruch 4 oder 5, wobei eine der beiden Komponenten (A) und (B) Dextranblau umfasst und/oder wobei eine der beiden Komponenten (A) und (B) Riboflavin umfasst,
vorzugsweise wobei die eine der beiden Komponenten (A) und (B) Dextranblau und die andere der beiden Komponenten (A) und (B) Riboflavin umfasst.

7. Sterilisierte, gelbildende Zusammensetzung nach einem der Ansprüche 4 bis 6,
wobei in einer der beiden Komponenten (A) und (B) Dextranblau in einem Bereich von 0,01 bis 1,0 Gew.-%, vorzugsweise 0,05 bis 0,75 Gew.-%, besonders bevorzugt 0,1 bis 0,5 Gew.-%, bezogen auf das Gewicht des Bestandteils (ii) bzw. des Bestandteils (b), vorliegt, und/oder
wobei in einer der beiden Komponenten (A) und (B) Riboflavin in einem Bereich von 0,0001 bis 0,05 Gew.-%, vorzugsweise 0,0005 bis 0,01 Gew.-%, besonders bevorzugt 0,001 bis 0,005 Gew.-%, bezogen auf das Gewicht des Bestandteils (ii) bzw. des Bestandteils (b), vorliegt.

8. Sterilisierte, gelbildende Zusammensetzung nach einem der vorangehenden Ansprüche, wobei die Komponente (A) und/oder die Komponente (B) und/oder eine weitere, gegebenenfalls in der Zusammensetzung enthaltene Komponente (C) einen neutralen pH-Wert aufweist, vorzugsweise einen pH-Wert im Bereich von 6,5 bis 8, besonders bevorzugt einen pH-Wert im Bereich von 7,0 bis 7,5.

9. Sterilisierte, gelbildende Zusammensetzung nach einem der vorangehenden Ansprüche,
wobei die Komponente (A), die Komponente (B) und/oder eine weitere, gegebenenfalls in der Zusammensetzung enthaltene Komponente (C) eine oder mehrere Substanzen zum Verbessern der Vernetzung und/oder der Stabilität des Natriumalginats enthält, insbesondere Quervernetzer, vorzugsweise wobei die, eine mehrere oder alle Substanz(en) ausgewählt ist/sind aus der Gruppe bestehend aus Aminosäuren, (Bio)Polymeren, Zuckerpolymeren, synthetischen Di- oder Multimeren, Zucker-Präpolymeren und synthetischen Präpolymeren,
und/oder
wobei die Komponente (A), die Komponente (B) und/oder eine weitere, gegebenenfalls in der Zusammensetzung enthaltenen Komponente (C) eine oder mehrere Substanzen zum Erhöhen der Dichte des vernetzbaren Polymers aus Bestandteil (i) der Komponente (A), vorzugsweise des Natriumalginats, enthält, vorzugsweise wobei die, eine mehrere oder alle Substanz(en) ausgewählt ist/sind aus der Gruppe bestehend aus (Bio)Polymeren, Zuckerpolymeren, synthetische Polymere, Zucker-Präpolymere, Zuckermonomere, Zuckerdimere wie Saccharose oder Glucose, synthetische Präpolymere, hydrophile Copolymere, Epichlorhydrin oder Glycerin.

10. Sterilisierte, gelbildende Zusammensetzung nach einem der vorangehenden Ansprüche, zur Anwendung in einem Verfahren zum Entfernen von unerwünschten Partikeln aus einem Patienten,
vorzugsweise wobei die Partikel Ablagerungen, Ausfallprodukte, Fremdkörper und/oder Fragmente davon und/oder Splitter körpereigener Strukturen sind.

11. Sterilisierte, gelbildende Zusammensetzung nach einem der vorangehenden Ansprüche, zur Anwendung nach Anspruch 10, wobei das Verfahren die folgenden Schritte umfasst:
(i) Bereitstellen der sterilisierten Komponenten (A) und (B),
(ii) Einbringen der Komponente (A) und (B) in den Körper des Patienten in einen Bereich, der zu entfernende Partikel enthält,
unter Bedingungen, die bei Kontakt der Komponente (A) mit der Komponente (B) eine Vernetzung des bzw. der vernetzbaren Polymere ermöglichen, so dass ein vernetztes Gel entsteht, das die zu entfernenden Partikel teilweise oder vollständig umschließt,
(iii) Entfernen des vernetzten Gels samt der davon umschlossenen Partikel aus dem Bereich des Körpers des Patienten.

12. Sterilisierte, gelbildende Zusammensetzung nach einem der vorangehenden Ansprüche, zur Anwendung nach Anspruch 10 oder 11,
wobei das Verfahren den folgenden weiteren Schritt umfasst, der zeitlich vor Schritt (ii) erfolgt:
Fragmentieren eines oder mehrerer Partikel in dem Bereich des Körpers des Patienten, so dass zwei oder mehrere, vorzugsweise eine Vielzahl von Fragmenten des/der Partikel(s) entsteht bzw. entstehen.

13. Verfahren zur Herstellung einer sterilisierten, gelbildenden Zusammensetzung nach einem der vorangehenden Ansprüche, umfassend oder bestehend aus den Schritten:
(i) Bereitstellen der Komponente (A), wie in einem der vorangehenden Ansprüche definiert,
(ii) Bereitstellen der Komponente (B), wie in einem der vorangehenden Ansprüche definiert,
(iii) optional: Bereitstellen einer Komponente (C), wie in Anspruch 9 definiert,
(iv) Sterilisieren der in den vorangehenden Schritten bereitgestellten Komponente (A) und optional Komponente (B) und/oder Komponente (C).

14. Verfahren nach Anspruch 13, wobei Schritt (iv) einen Schritt der Dampfsterilisation enthält oder daraus besteht,
vorzugsweise wobei die Temperatur bei der Dampfsterilisation mindestens 121 °C beträgt, besonders vorzugsweise in einem Bereich von 121 °C bis 134 °C liegt,
vorzugsweise wobei der Druck bei der Dampfsterilisation mindestens 2 bar beträgt, besonders vorzugsweise in einem Bereich von 2 bis 3 bar liegt,
und vorzugsweise wobei die Dauer der Dampfsterilisation mindestens 5 Minuten beträgt, besonders vorzugsweise in einem Bereich von 5 bis 25 Minuten liegt.

15. Sterilisierte, gelbildende Zusammensetzung nach einem der Ansprüche 1 bis 12, hergestellt oder herstellbar durch ein Verfahren nach Anspruch 14.

## Claims

1. Sterilized, gel-forming multi-component composition, preferably two-component composition, for forming a gel,
the composition comprising or consisting of
component (A), containing
(i) one or more cross-linkable, preferably cationically cross-linkable, polymer(s), wherein the or one polymer is sodium alginate,
(ii) water,
(iii) NaCl in a range of from 0.3 to 1.2 wt.-%, based on the total weight of component (A),
(iv) one or more phosphate buffer(s)
and
component (B), containing
(a) one or more cross-linking agent(s) for cross-linking the cross-linkable polymer(s) in component (i) of component (A), preferably wherein the or one cross--linking agent is CaCl₂, and
(b) water.

2. Sterilized, gel-forming multi-component composition according to claim 1, wherein the phosphate buffer in component (A) contains one or more phosphate(s) selected from the group consisting of H₃PO₄, H₂PO₄⁻, HPO₄²-, PO₄³⁻, and their salts, preferably sodium and potassium salts, and mixtures of the phosphates and their salts, preferably sodium and potassium salts.

3. Sterilized, gel-forming composition according to any one of the preceding claims,
wherein in component (A)
sodium alginate (component (i)) is present in a range of from 0.5 to 10 wt.%, preferably 0.5 to 7.5 wt.-%, further preferably 0.5 to 5 wt.-%, even further preferably 0.5 to 2.5 wt.-%, particularly preferably 0.5 to 1.25 wt.-%, especially preferably 0.75 to 1.0 wt.-%, based on the weight of component (ii),
and/or
wherein in component (B)
CaCl₂ (component (a)) is present in a range of from 0.25 to 5.0 wt.-%, preferably 0.75 to 2.5 wt.-%, further preferably 1.5 to 2.0 wt.-%, based on the weight of compound (b).

4. Sterilized, gel-forming composition according to any one of the preceding claims, wherein component (A) and/or component (B) comprise(s) at least one dye.

5. Sterilized, gel-forming composition according to claim 4, wherein component (A) and component (B) comprise at least one dye and wherein the dye(s) in component (A) is/are different from the dye(s) in component (B).

6. Sterilized, gel-forming composition according to claim 4 or 5, wherein one of components (A) and (B) comprises dextran blue and/or wherein one of components (A) and (B) comprises riboflavin,
preferably wherein one of the components (A) and (B) comprises dextran blue and the other of the components (A) and (B) comprises riboflavin.

7. Sterilized, gel-forming composition according to any one of claims 4 to 6,
wherein in one of the components (A) and (B) dextran blue is present in a range of from 0.01 to 1.0 wt.-%, preferably 0.05 to 0.75 wt.-%, particularly preferably 0.1 to 0.5 wt.-%, based on the weight of component (ii) or, respectively, of component (b), and/or
wherein in one of the components (A) and (B) riboflavin is present in a range of from 0.0001 to 0.05 wt.-%, preferably 0.0005 to 0.01 wt.-%, particularly preferably 0.001 to 0.005 wt.-%, based on the weight of component (ii) or, respectively, of component (b).

8. Sterilized, gel-forming composition according to any one of the preceding claims, wherein component (A) and/or component (B) and/or a further component (C), which may also be present in the composition, has a neutral pH, preferably a pH in the range of from 6.5 to 8, particularly preferably a pH in the range of from 7.0 to 7.5.

9. Sterilized, gel-forming composition according to any one of the preceding claims
wherein component (A), component (B), and/or a further component (C), which may also be present in the composition, contains one or more substances for improving the cross-linking and/or the stability of the sodium alginate, particularly cross-linkers, preferably wherein the, one or more or all substance(s) is/are selected from the group consisting of amino acids, (bio)polymers, sugar polymers, synthetic di- or mul-timers, sugar-pre-polymers, and synthetic pre-polymers,
and/or
wherein component (A), component (B), and/or a further component (C), which may also be present in the composition, contains one or more substances for increasing the density of the cross-linkable polymer of component (i) of component (A), preferably of the sodium alginate, preferably wherein the, one or more or all substance(s) is/are selected from the group consisting of (bio)polymers, sugar polymers, synthetic polymers, sugar-pre-polymers, sugar monomers, sugar dimers such as sucrose or glucose, synthetic pre-polymers, hydrophilic co-polymers, epichlorohydrin or glycerol.

10. Sterilized, gel-forming composition according to any one of the preceding claims, for use in a method for removing undesired particles from a patient,
preferably wherein the particles are deposits, precipitation products, foreign particles and/or fragments thereof and/or splinters of endogenous structures.

11. Sterilized, gel-forming composition according to any one of the preceding claims, for use according to claim 10, wherein the method comprises the following steps:
(i) providing the sterilized components (A) and (B),
(ii) introducing components (A) and (B) into the body of the patient in an area comprising particles to be removed,
under conditions allowing a cross-linking of the cross-linkable polymer(s) under contact of component (A) with component (B), such that a cross-linked gel is formed, which partially or fully encloses the particle(s) to be removed,
(iii) removing the cross-linked gel including the particles enclosed therein from the area of the body of the patient.

12. Sterilized, gel-forming composition according to any one of the preceding claims, for use according to claim 10 or 11,
wherein the method further comprises the step, which is performed temporally before step (ii):
fragmenting one or more particle(s) in the area of the body of the patient, such that two or more, preferably a plurality of fragments of the particle(s) is/are obtained.

13. Method for producing a sterilized, gel-forming composition according to one or the preceding claims, comprising or consisting of the steps:
(i) providing component (A), as defined in one of the preceding claims,
(ii) providing component (B), as defined in one of the preceding claims,
(iii) optionally: providing a component (C), as defined in claim 9,
(iv) sterilizing the component (A) and optionally component (B) and/or (C), which was/were provided in one of the preceding steps.

14. Method according to claim 13, wherein step (iv) comprises or consists of a step of steam sterilization,
preferably wherein the temperature of the steam sterilization is at least 121 °C, particularly preferably is in the range of from 121 °C to 134 °C,
preferably wherein the pressure during the steam sterilization is at least 2 bar, particularly preferably is in a range of from 2 to 3 bar,
and preferably wherein the duration of the steam sterilization is at least 5 minutes, particularly preferably is in a range of from 5 to 25 minutes.

15. Sterilized, gel-forming composition according to one of claims 1 to 12, obtained or obtainable by a method according to claim 14.

## Revendications

1. Composition multicomposante gélifiante stérilisée, de préférence composition bi-composante, destinée à former un gel,
la composition consistant en ou comprenant
le composant (A), contenant
(i) un ou plusieurs polymère(s) réticulables, de préférence réticulables cationiquement, dans laquelle le ou un polymère est l'alginate de sodium,
(ii) de l'eau,
(iii) du NaCl dans une plage allant de 0,3 à 1,2 % en poids, sur la base du poids total du composant (A),
(iv) un ou plusieurs tampon(s) phosphate
et
le composant (B), contenant
(a) un ou plusieurs agent(s) de réticulation pour réticuler le ou les polymère(s) réticulable(s) dans le constituant (i) du composant (A), de préférence dans laquelle le ou un agent de réticulation est du CaCl₂, et
(b) de l'eau.

2. Composition gélifiante stérilisée selon la revendication 1, dans laquelle le tampon phosphate dans le composant (A) contient un ou plusieurs phosphate(s) qui sont choisi(s) dans le groupe constitué de H₃PO₄, H₂PO₄⁻, HPO₄²⁻, PO₄³⁻, ainsi que leurs sels, de préférence les sels de sodium et de potassium, et des mélanges de phosphates et de leurs sels, de préférence les sels de sodium et de potassium.

3. Composition gélifiante stérilisée selon l'une quelconque des revendications précédentes,
dans laquelle dans le composant (A)
l'alginate de sodium (constituant (i)) est présent dans une plage allant de 0,5 à 10 % en poids, de préférence de 0,5 à 7,5 % en poids, de manière particulièrement préférée de 0,5 à 5 % en poids, plus préférablement de 0,5 à 2,5 % en poids, en outre de préférence de 0,5 à 1,25 % en poids; de manière particulièrement préférée de 0,75 à 1,0 % en poids, par rapport au poids du constituant (ii),
et/ou
dans laquelle dans le composant (B)
du CaCl₂ (constituant (a)) est présent dans une plage allant de 0,25 à 5,0 % en poids, de préférence de 0,75 à 2,5 % en poids, de manière particulièrement préférée de 1,5 à 2,0 % en poids, par rapport au poids du constituant (b).

4. Composition gélifiante stérilisée selon l'une quelconque des revendications précédentes, dans laquelle le composant (A) et/ou le composant (B) comprend/comprennent au moins un colorant.

5. Composition gélifiante stérilisée selon la revendication 4, dans laquelle le composant (A) et le composant (B) comprennent au moins un colorant et dans laquelle le/les colorant(s) dans le composant (A) est/sont différent(s) du/des colorant(s) dans le composant (B).

6. Composition gélifiante stérilisée selon la revendication 4 ou 5, dans laquelle l'un des deux composants (A) et (B) comprend du bleu de dextrane et/ou dans laquelle l'un des deux composants (A) et (B) comprend de la riboflavine,
de préférence dans laquelle l'un des deux composants (A) et (B) comprend du bleu de dextrane et l'autre des deux composants (A) et (B) comprend de la riboflavine.

7. Composition gélifiante stérilisée selon l'une quelconque des revendications 4 à 6,
dans laquelle dans l'un des deux composants (A) et (B) du bleu de dextrane est présent dans une plage allant de 0,01 à 1,0 % en poids, de préférence de 0,05 à 0,75 % en poids, de manière particulièrement préférée de 0,1 à 0,5 % en poids, par rapport au poids du constituant (ii) ou bien du constituant (b), et/ou
dans laquelle dans l'un des deux composants (A) et (B) la riboflavine est présente dans une plage allant de 0,0001 à 0,05 % en poids, de préférence de 0,0005 à 0,01 % en poids, de manière particulièrement préférée de 0,001 à 0,005 % en poids, par rapport au poids du constituant (ii) ou bien du constituant (b).

8. Composition gélifiante stérilisée selon l'une quelconque des revendications précédentes, dans laquelle le composant (A) et/ou le composant (B) et/ou un autre composant (C) contenu le cas échéant dans la composition présente une valeur pH neutre, de préférence une valeur pH comprise entre 6,5 et 8, de manière particulièrement préférée une valeur pH comprise entre 7,0 et 7,5.

9. Composition gélifiante stérilisée selon l'une quelconque des revendications précédentes,
dans laquelle le composant (A), le composant (B) et/ou un autre composant (C) contenu le cas échéant dans la composition contient une ou plusieurs substance(s) destinée(s) à améliorer la réticulation et/ou la stabilité de l'alginate de sodium, en particulier des agents de cross-linking, de préférence dans laquelle la, une, plusieurs ou toutes les substances est/sont choisie(s) dans le groupe constitué d'acides aminés, de (bio)polymères, de polymères de sucre, de di- ou multimères synthétiques, de prépolymères de sucre et de prépolymères synthétiques,
et/ou
dans laquelle le composant (A), le composant (B) et/ou un autre composant (C) contenu le cas échéant dans la composition, contient une ou plusieurs substance(s) destinée(s) à augmenter la densité du polymère réticulable du constituant (i) du composant (A), de préférence de l'alginate de sodium, de préférence dans laquelle la, une, plusieurs ou toutes les substance(s) est/sont choisie(s) dans le groupe constitué des (bio)polymères, des polymères de sucre, des polymères synthétiques, des prépolymères de sucre, des monomères de sucre, des dimères de sucre tels que le saccharose ou le glucose, des prépolymères synthétiques, des copolymères hydrophiles, de l'épichlorhydrine ou de la glycérine.

10. Composition gélifiante stérilisée selon l'une quelconque des revendications précédentes, destinée à être utilisée dans un procédé d'élimination de particules indésirables d'un patient,
de préférence dans laquelle les particules sont des dépôts, des produits de précipitation, des corps étrangers et/ou des fragments de ceux-ci et/ou des éclats de propres structures du corps.

11. Composition gélifiante stérilisée selon l'une quelconque des revendications précédentes, pour une utilisation selon la revendication 10, dans laquelle le procédé comprend les étapes suivantes consistant à:
(i) fournir les composants stérilisés (A) et (B),
(ii) introduire les composants (A) et (B) dans le corps du patient dans une zone qui contient des particules à éliminer,
dans des conditions qui, lorsque le composant (A) entre en contact avec le composant (B), permettent une réticulation du ou bien des polymère(s) réticulable(s), de sorte qu'il se forme un gel réticulé qui entoure en partie ou complètement les particules à éliminer,
(iii) éliminer le gel réticulé, y compris les particules qu'il entoure, de la zone du corps du patient.

12. Composition gélifiante stérilisée selon l'une quelconque des revendications précédentes, pour une utilisation selon la revendication 10 ou 11,
dans lequel le procédé comprend l'étape supplémentaire suivante qui a lieu avant l'étape (ii):
fragmenter une ou plusieurs particule(s) dans la zone du corps du patient de sorte que deux ou plusieurs, de préférence une pluralité de fragments de la ou des particule(s) soit ou bien soient créé(s).

13. Procédé de production d'une composition gélifiante stérilisée selon l'une quelconque des revendications précédentes, comprenant ou consistant en les étapes:
(i) fournir le composant (A), tel que défini dans l'une quelconque des revendications précédentes,
(ii) fournir le composant (B), tel que défini dans l'une quelconque des revendications précédentes,
(iii) en option: fournir un composant (C) tel que défini dans la revendication 9,
(iv) stériliser le composant (A) et en option le composant (B) et/ou le composant (C) fournis dans les étapes précédentes.

14. Procédé selon la revendication 13, dans lequel l'étape (iv) contient ou consiste en une étape de stérilisation à la vapeur,
de préférence, dans lequel la température lors la stérilisation à la vapeur est d'au moins 121 °C, de manière particulièrement préférée se situe dans une plage allant de 121 °C à 134 °C,
de préférence, dans lequel la pression lors de la stérilisation à la vapeur est d'au moins 2 bars, de manière particulièrement préférée se situe dans une plage allant de 2 à 3 bars,
et, de préférence dans lequel la durée de la stérilisation à la vapeur est d'au moins 5 minutes, de manière particulièrement préférée se situe dans une plage allant de 5 à 25 minutes.

15. Composition gélifiante stérilisée selon l'une quelconque des revendications 1 à 12, produite ou apte à être produite par un procédé selon la revendication 14.
